# EUROPÄISCHE PATENTSCHRIFT

(11) **EP 0 408 857 B1**
(45) Veröffentlichungstag und Bekanntmachung des Hinweises auf die Patenterteilung: **29.09.1993**
(21) Anmeldenummer: 90109418.5
(22) Anmeldetag: 18.05.1990
(51) Int. Cl.: C07C 217/08, C07C 69/734, C08G 65/26, C07C 213/04, C07C 67/26

(54) **Verfahren zur Herstellung von alkylenoxid- und dioxan-1,4-armen nicht-ionogenen Tensiden mittels Alkalimetallalkoholat als Katalysator**
Process for the preparation of nonionic surfactants low in alkylene oxides and 1,4-dioxane by means of alkali metal alcoholate catalyst
Procédé pour la préparation de tensio-actifs non-ioniques ayant une faible concentration en oxydes d'alkylènes au moyen d'un alcoolat d'un métal alcalin comme catalyseur

(30) Priorität: 17.07.1989 DE 3923563
(43) Veröffentlichungstag der Anmeldung: 23.01.1991
(73) Patentinhaber: HÜLS AKTIENGESELLSCHAFT, 45764 Marl (DE)
(72) Erfinder: Schröer, Egbert, D-4270 Dorsten (DE); Schulze, Klaus, Dr., D-4358 Haltern (DE); Wienhöfer, Ekkehard, Dr., D-4370 Marl (DE)

(56) Entgegenhaltungen:
- EP-A- 0 026 547
- EP-A- 0 115 083
- DE-B- 1 132 123
- FR-A- 1 116 930
- US-A- 4 453 023

## Beschreibung

Die Erfindung betrifft die Herstellung von alkylenoxid- und dioxan-1,4-armen nicht-ionogenen Tensiden durch Katalyse mit einem Alkalimetallfettalkoholat sowie die Herstellung des Alkalimetallfettalkoholates.

Aufgrund der meist täglichen Anwendung von Produkten, in denen nicht-ionogene Tenside enthalten sind und auf dem Hintergrund möglicher toxikologischer Gefährdungen durch größere Restmengen an Alkylenoxiden und Dioxan-1,4 in diesen Tensiden, ist es notwendig, alkylenoxid- und dioxan-1,4-arme Produkte zur Verfügung zu stellen.

Üblicherweise erfolgt die Herstellung nicht-ionogener Tenside durch Katalyse mit Na- bzw. K-Ionen, welche vorzugsweise als wäßrige NaOH oder KOH bzw. als Na- oder K-methylat den Edukten wie Alkylphenolen, Fettalkoholen, Glycolen, Aminen, Fettsäuren und Ölen zugesetzt werden. Lösungs- und Reaktionswasser werden nach der Inertisierung mit Stickstoff entfernt. Anschließend erfolgt die Umsetzung mit Alkylenoxid (FR-A- 1116930).

Nach der US-PS 4 453 023 können auch Ba-Alkoholate als Katalysator dienen, die nach dieser US-Druckschrift durch Reaktion des Ba-Metalles mit Ethanol und anschließender Umsetzung z. B. mit 2-Ethylhexanol hergestellt werden können. Das entstehende Bariumethylhexanolat wird durch Vakuumdestillation von Ethanol gereinigt.

Eine ähnliche Katalysatorherstellung beschreibt die EP-PS 0 026 547. Hiernach können Ca-, Sr- oder Ba-Alkoholate durch Reaktion des Ca-, Sr- oder Ba-Metalles mit Ethanol und anschließender Umsetzung mit Decanol hergestellt werden. Das dabei wieder entstehende Ethanol wird nachher durch Anlegen von Vakuum entfernt.

Sowohl das Verfahren zur Herstellung von nicht-ionogenen Tensiden nach der US-PS 4 453 023 als auch der Einsatz von Katalysatoren bei der Herstellung nicht-ionogener Tenside, die nach dem zweistufigen Verfahren durch Umsetzung eines Alkalimetalles mit Ethanol und anschließender Umsetzung dieses Ethanolats mit einem höheren Alkohol hergestellt worden sind, weisen neben einer extremen Kostenungünstigkeit den Nachteil hoher Restmengen an Alkylenoxid (≈ 200 ppm) und Dioxan-1,4 (≈ 5 000 ppm) auf. Außerdem ist der Einsatz von Metallen zur Alkoholatherstellung aufgrund der Wasserstoffentwicklung nicht unproblematisch und Produkte, die mit Ethylhexanolatkatalysatoren hergestellt worden sind, riechen stark nach Ethylhexanol.

Es bestand daher die Aufgabe, ein Verfahren zur Herstellung alkylenoxid- und dioxan-1,4-armer, nicht-ionogener, oxethylierter Tenside aufzufinden.

Die Aufgabe wurde durch ein Verfahren, das bei der Alkoxylierung der Edukte als Katalysator eine durch direkte Umsetzung von Alkalihydroxid mit Fettalkohol gewonnenes Fettalkoholat verwendet, gelöst.

Gegenstand der Erfindung ist daher ein Verfahren zur Herstellung alkylenoxid- und dioxan-1,4-armer Alkoxidationsprodukte von Aminen, Fettsäuren und Ölen, das dadurch gekennzeichnet ist, daß man als Katalysator durch direkte Umsetzung bei erhöhter Temperatur von Alkalihydroxid mit Fettalkoholen gewonnene Alkalifettalkoholate einsetzt.

Es wurde überraschend gefunden, daß sich durch den Einsatz von erfindungsgemäß hergestellten Katalysatoren der Alkylenoxid-Gehalt in den nicht-ionogenen Tensiden auf unter 10 ppm und der Dioxan-1,4-Gehalt auf unter 100 ppm senken läßt.

Als Fettalkohol zur Herstellung des Katalysators wird bevorzugt Isotridecanol verwendet, das bevorzugt mit Kalium- oder Natriumhydroxid bei Temperaturen von 100 - 160 °C, bevorzugt 120 - 140 °C, umgesetzt wird.

Die bevorzugt zur Alkoxilierung verwendeten Edukte sind Öle, wie Ricinusöl, Kokosöl, Sojaöl und C₈ bis C₂₄ Mono-/diglycerid-Gemische, Fettsäuren von C₁₂ bis C₂₂ und Amine mit C₁₂ bis C₂₄, bevorzugt C₁₂ bis C₁₈.

Die Temperaturen betragen hierbei etwa 140 - 200 °C, bevorzugt 160 - 190 °C.

Die folgenden Beispiele sollen die Erfindung verdeutlichen.

### Beispiel 1 (erfindungsgemäß)

a) In einem 5-l Glasreaktor mit Heizung, Rührer und Stickstoffeinleitung werden 561 g Kaliumhydroxid (Plätzchen) und 2 029 g i-Tridecanol auf 130 °C unter Rühren aufgeheizt und 48 Stunden bei dieser Temperatur gerührt. Entstehendes Wasser wird mit Hilfe von Stickstoff ausgetrieben.
   Das entstehende Alkoholat ist bei 70 - 90 °C pump- und fließfähig.
b) 561 g 50%ige KOH und 1 014,5 g i-Tridecanol werden bei 60 °C gemischt. Anschließend wird die Emulsion mit 500 g/h Zutropfgeschwindigkeit bei 130 °C unter Stickstoffabdeckung über einen Dünnschichtverdampfer zur Reaktion gebracht und Wasser abgetrennt. Es entsteht ein bei 70 - 90 °C fließ- und pumpfähiges Alkoholat.

### Beispiel 2 (erfindungsgemäß)

a) In der Apparatur und unter den Bedingungen von Beispiel 1a werden 400 g Natriumhydroxid (Plätzchen) und 2 029 g i-Tridecanol vorgelegt. Der Ansatz wird 72 Stunden unter Rühren bei 130 °C belassen. Entstehendes Wasser wird mit Hilfe von Stickstoff ausgetrieben. Das Alkoholat ist bei 70 - 90 °C fließ- und pumpfähig.
b) Am Dünnschichtverdampfer werden unter den Bedingungen von Beispiel 1b aus 800 g 25%iger Natriumhydroxid-Lösung und 1 014,5 g i-Tridecanol ein Natriumisotridecanolat hergestellt, das bei 70 - 90 °C fließ- und pumpfähig ist.

### Beispiel 3 (erfindungsgemäß)

In einem 5-l Reaktor mit Heizung, Rührer und Stickstoffeinleitung werden 370 g (2 mol) Laurylamin auf 60 °C aufgeheizt und unter Stickstoffeinleitung zur Inertisierung verrührt. Anschließend werden bei 160 °C unter 0,5 bar Stickstoffvordruck und einem Maximaldruck von 3,5 bar 176 g (4 mol) Ethylenoxid angelagert. Nachdem der Ansatz auf 60 °C abgekühlt ist, werden 2,42 g (10,16 mmol) K-isotridecanolat zugegeben und unter Stickstoffeinleitung zur Inertisierung verrührt. Anschließend werden bei 160 °C unter 1,5 bar abs Stickstoffvordruck und bei einem Maximaldruck von 3 bar abs 704 g (16 mol) Ethylenoxid angelagert.
- Rest Ethylenoxid-Gehalt: : < 1 ppm
- Dioxan-1,4 Gehalt: : 75 ppm

### Beispiel 4 (erfindungsgemäß)

In der Apparatur und unter den Bedingungen von Beispiel 3 werden 566,7 g (2 mol) Ölsäure und 3,58 g (15,05 mmol) Na-isotridecanolat auf ca. 60 °C aufgeheizt und unter Stickstoffeinleitung zur Inertisierung verrührt. Anschließend werden bei 180 °C 616 g (14 mol) Ethylenoxid angelagert.
- Rest Ethylenoxid-Gehalt: : 5 ppm
- Dioxan-1,4 Gehalt: : 80 ppm

### Beispiel 5 (erfindungsgemäß)

In der Apparatur und unter den Bedingungen von Beispiel 3 werden 936 g (1 mol) Ricinusöl und 8,16 g (34,27 mmol) Na-isotridecanolat auf ca. 60 °C aufgeheizt und unter Stickstoffeinleitung zur Inertisierung verrührt. Anschließend werden bei 180 °C 1 760 g (40 mol) Ethylenoxid angelagert.
- Rest Ethylenoxid-Gehalt: : 6 ppm
- Dioxan-1,4 Gehalt: : 80 ppm

### Beispiel 6 (Vergleichsbeispiel)

Wie in Beispiel 3 werden 370 g (2 mol) Laurylamin auf 60 °C aufgeheizt und unter Stickstoffeinleitung zur Inertisierung verrührt. Anschließend werden bei 160 °C 176 g (4 mol) Ethylenoxid angelagert. Nachdem der Ansatz auf 60 °C abgekühlt ist, werden 10,16 mmol Ba-ethylhexanolat, hergestellt nach dem in US-PS 4 453 023 beschriebenen Verfahren, zugegeben und unter Stickstoffeinleitung zur Inertisierung verrührt. Anschließend werden bei 160 °C 704 g (16 mol) Ethylenoxid angelagert.
Das Produkt riecht stark nach Ethylhexanol.
- Rest Ethylenoxid-Gehalt: : 120 ppm
- Dioxan-1,4 Gehalt: : 890 ppm

### Beispiel 7 (Vergleichsbeispiel)

Wie in Beispiel 4 werden 566,7 g (2 mol) Ölsäure und 15,05 mmol Ba-Decanolat, hergestellt nach dem in EP 0 026 547 beschriebenen Verfahren, auf ca. 60 °C aufgeheizt und unter Stickstoffeinleitung zur Inertisierung verrührt. Anschließend werden bei 180 °C 616 g (14 mol) Ethylenoxid angelagert.
- Rest Ethylenoxid-Gehalt: : 150 ppm
- Dioxan-1,4 Gehalt: : 4 700 ppm

### Beispiel 8 (Vergleichsbeispiel)

Wie in Beispiel 5 werden 936 g (1 mol) Ricinusöl und 34,27 mmol 25%ige NaOH verrührt und Wasser durch Einleitung von Stickstoff bei 130 °C ausgetrieben. Anschließend werden bei 180 °C 1 760 g (40 mol) Ethylenoxid angelagert.
- Rest Ethylenoxid-Gehalt: : 200 ppm
- Dioxan-1,4 Gehalt: : 2 600 ppm

## Patentansprüche

1. Verfahren zur Herstellung von alkylenoxid- und dioxan-1,4-armen Alkoxidationsprodukten von Aminen, Fettsäuren und Ölen,
dadurch gekennzeichnet,
daß man als Katalysator durch direkte Umsetzung bei erhöhten Temperaturen von Alkalihydroxid mit Fettalkoholen gewonnene Alkalifettalkoholate einsetzt.

2. Verfahren zur Herstellung von alkylenoxid- und dioxan-1,4-armen Alkoxidationsprodukten von Aminen, Fettsäuren und Ölen nach Anspruch 1,
dadurch gekennzeichnet,
daß man als Alkalihydroxid Natrium- oder Kaliumhydroxid verwendet.

3. Verfahren zur Herstellung von alkylenoxid- und dioxan-1,4-armen Alkoxidationsprodukten von Aminen, Fettsäuren und Ölen nach Anspruch 1,
dadurch gekennzeichnet,
daß man als Fettalkohol zur Katalysatorherstellung Isotridecanol verwendet.

4. Verfahren zur Herstellung von alkylenoxid- und dioxan-1,4-armen Alkoxidationsprodukten von Aminen, Fettsäuren und Ölen nach Anspruch 1,
dadurch gekennzeichnet,
daß man die Umsetzung bei Temperaturen von 100 - 160 °C durchführt.

5. Verfahren zur Herstellung von alkylenoxid- und dioxan-1,4-armen Alkoxidationsprodukten von Aminen, Fettsäuren und Ölen nach Anspruch 4,
dadurch gekennzeichnet,
daß die Temperatur 120 - 140 °C beträgt.

## Revendications

1. Procédé de préparation de produits, pauvres en oxyde d'alkylène et en dioxane-(1,4), d'amines, d'acides gras et d'huiles,
caractérisé par le fait que l'on utilise comme catalyseur des alcoolates alcalins gras qui sont obtenus en faisant directement réagir à des température élevées un hydroxyde alcalin sur des alcools gras.

2. Procédé de préparation de produits, pauvres en oxyde d'alkylène et en dioxane-(1,4), d'amines, d'acides gras et d'huiles selon la revendication 1,
caractérisé par le fait que l'on utilise l'hydroxyde de sodium ou l'hydroxyde de potassium comme hydroxyde alcalin.

3. Procédé de préparation de produits, pauvres en oxyde d'alkylène et en dioxane-(1,4), d'amines, d'acides gras et d'huiles selon la revendication 1,
caractérisé par le fait que l'on utilise l'iso-tridécanol en tant qu'alcool gras pour la préparation du catalyseur.

4. Procédé de préparation de produits, pauvres en oxyde d'alkylène et en dioxane-(1,4), d'amines, d'acides gras et d'huiles selon la revendication 1,
caractérisé par le fait que l'on effectue la réaction à des températures de 100 à 160°C.

5. Procédé de préparation de produits, pauvres en oxyde d'alkylène et en dioxane-(1,4), d'amines, d'acides gras et d'huiles selon la revendication 4,
caractérisé par le fait que la température est de 120 à 140°C.

## Claims

1. A process for the preparation of products of the alkoxidation of amines, fatty acids and oils, which products have a low alkylene oxide and 1,4-dioxane content, characterised in that the catalyst employed is an alkali metal fatty alcoholate obtained by direct reaction of alkali metal hydroxide with fatty alcohols at elevated temperature.

2. A process for the preparation of products of the alkoxidation of amines, fatty acids and oils according to claim 1, which products have a low alkylene oxide and 1,4-dioxane content, characterised in that the alkali metal hydroxide used is sodium hydroxide or potassium hydroxide.

3. A process for the preparation of products of the alkoxidation of amines, fatty acids and oils according to claim 1, which products have a low alkylene oxide and 1,4-dioxane content, characterised in that the fatty alcohol used for the preparation of the catalyst is isotridecanol.

4. A process for the preparation of products of the alkoxidation of amines, fatty acids and oils according to claim 1, which products have a low alkylene oxide and 1,4-dioxane content, characterised in that the reaction is carried out at temperatures of from 100 to 160°C.

5. A process for the preparation of products of the alkoxidation of amines, fatty acids and oils according to claim 1, which products have a low alkylene oxide and 1,4-dioxane content, characterised in that the temperature is from 120 to 140°C.
